# EUROPÄISCHE PATENTSCHRIFT

(11) **EP 0 267 408 B1**
(45) Veröffentlichungstag und Bekanntmachung des Hinweises auf die Patenterteilung: **10.03.1993**
(21) Anmeldenummer: 87114318.6
(22) Anmeldetag: 01.10.1987
(51) Int. Cl.: C12M 1/02, C12M 3/00, B01F 7/26

(54) **Bioreaktor zum Kultivieren von biologischem Material**
Bioreactor for cultivating biological material
Bioréacteur pour culture de matériau biologique

(30) Priorität: 08.10.1986 DE 3634203
(43) Veröffentlichungstag der Anmeldung: 18.05.1988
(73) Patentinhaber: BOEHRINGER MANNHEIM GMBH, 68298 Mannheim (DE)
(72) Erfinder: Kearns, Michael John, Dr., D-8124 Seeshaupt (DE); Comer, Michael James, Dr., D-8139 Bernried (DE)

(56) Entgegenhaltungen:
- CH-A- 376 089
- DE-B- 1 258 837
- FR-A- 1 133 188
- GB-A- 938 873
- US-A- 4 004 786

## Beschreibung

Die Erfindung betrifft einen Bioreaktor zum Kultiviern von vermehrungsfähigem biologischem Material, insbesondere von auf Mikrocarriern haftenden adherenten Zellen, mit einem Gefäß für ein Kulturmedium mit Einrichtungen zur Kontrolle der Umweltbedingungen in dem Kulturmedium und einer Rühreinrichtung zur homogenen Verteilung der Zellen in dem Kulturmedium, welche einen Rotationsantrieb und eine im Inneren des Gefäßes verlaufende Rotationsachse aufweist.

Bei der biotechnologischen Herstellung von Substanzen, insbesondere Proteinen für pharmazeutische oder medizinisch-diagnostische Zwecke werden die entsprechenden biologischen Materialien, aus denen die Produkte gewonnen werden, in einem Kulturmedium innerhalb eines entsprechenden Bioreaktors vermehrt. Diese werden häufig auch als Fermenter bezeichnet.

Das Biomaterial besteht üblicherweise aus Mikroorganismen oder aus tierischen, humanen, pflanzlichen Zellen oder aus Hybridzellen. Während die Mikroorganismen im allgemeinen kein Trägermaterial benötigen, können die Zellen in vielen Fällen nur auf einem geeigneten Trägermaterial kultiviert werden. Man spricht in diesem Fall von adherenten Zellen. Die Erfindung richtet sich insbesondere auf Zellkulturen, bei denen adherente Zellen auf sehr kleinen Kügelchen, beispielsweise aus Dextran, haften, die man als Mikrocarrier bezeichnet. Der Einfachheit halber wird im folgenden nur von der Kultivierung von Zellen gesprochen. Dadurch soll die allgemeine Anwendbarkeit der Erfindung auch für andere Biomaterialien jedoch nicht beschränkt werden.

Bioreaktoren haben Einrichtungen, um die Umweltbedingungen (beispielsweise Sauerstoffparticaldruck, Temperatur, Nährstoffkonzentration) zu kontrollieren und in einem Bereich zu halten, in dem sich die Kultur möglichst intensiv vermehrt. Diese Bedingungen sowie die Dichte der Zellen in des Kulturmedium sollen überall in dem Bioreaktor möglichst gleich sein, d. h. es ist für du Wachstum der Kultur sehr wichtig, daß eine homogene Verteilung der Zellen in der Kultur erreicht wird.

Zu dieses Zweck sind bereits Rühreinrichtungen verschiedener Art verwendet worden. Beispielsweise werden Schiffsschraubenrührer eingesetzt, die an einer im Inneren des Bioreaktor rotierenden Achse befestigt sind. Diese Rührer stellen zwar eine ausreichende Homogenität der Zellen in dem Kulturmedium sicher, wenn sie ausreichend schnell rotieren. Die verhältnismäßig schnelle Rotation bringt jedoch die Gefahr mit sich, daß empfindliche Zellen, insbesondere adherente Zellen auf Mikrocarriern, verletzt werden.

In der Europäischen Patentanmeldung mit der Publikationsnummer 53869 ist eine speziell für Mikrocarrierkulturen adherenter Zellen geeignete Rühreinrichtung beschrieben, bei der ein zentral aufgehängtes Rührpaddel in eines Fermentationsgefäß eine kreisförmig schwingende Bewegung macht. Derartige Paddelrührer sind jedoch nur für verhältnismäßig kleine Bioreaktoren geeignet.

Es besteht daher ein Bedürfnis nach einem Bioreaktor, der sich auch für vorhältnismäßig große Volumina an Zellkultur (mehr als 10 l) eignet und dessen Rühreinrichtung eine homogene Verteilung der Zellen in des Kulturmedium sicherstellt, ohne daß die Zellen beschädigt werden.

Dieses Bedürfnis wird von einem Bioreaktor zum Kultivieren von Biomaterial der eingangs bezeichneten Art erfüllt, dessen Rühreinrichtung mehrere an der Rotationsachse befestigte, zur Rotationsachse geneigte, flache Rührscheiben aufweist. Die Rüchrscheiben sind bevorzugt ebene, kreisrunde Platte, durch deren Zentrum die Rotationsachse läuft. Sie können aur ihrer Oberfläche hervorstehende Bauelemente oder Strukturen aufweisen, die bevorzugt jedoch so gestaltet sind, daß sie keine scharfen Kanten oder Ecken haben und keine Turbulenzen in des Kulturmedium erzeugen. Sie können auch in ihrer Gesamtheit gewellt sein oder in anderer Weise von einer völlig ebenen Oberflächengestaltung abweichen. Besonders bevorzugt ist jedoch eine glattflächige, weitgehend ebene Oberfläche auf beiden Seiten der Scheiben.

Die Stärke der Scheiben kann in verhältnismaßig weiten Bereichen variieren, sie muß jedoch flach sein in des sich, daß ihre Flächenausdehnung um ein vielfaches größer ist als ihre Materialstärke. Aus allgemeinen konstruktiven und aus Kostengründen wird man im allgemeinen eine Materialstärke wählen, die nicht wesentlich größer ist, als nach den gegebenen mechanischen Belastungen erforderlich.

Die Verwendung von mindestens einer zur Rotationsachse geneigten flachen Scheibe als Misch- oder Knetvorrichtung, ist aus der GB-A-938 873, CH-A-376 089, DE-B-12 58 837 und FR-A-1133 188 bekannt. Ein Hinweis auf die Kultivierung von Zellen ist jedoch keiner dieser Druckschriften zu entnehmen.

In einem erfindungsgemäßen Bioreaktor wird eine sehr schnelle und vollständige Homogenisierung der Kultur bereits mit sehr geringen Umdrehungszahlen von weniger als 50 pro Minute, vorzugsweise weniger als 35 pro Minute, besonders bevorzugt weniger als 20 pro Minute, erreicht. Gleichzeitig wird das Biomaterial sehr schonend behandelt, so daß auch empfindliche Zellen nicht beschädigt werden.

Ein besonders guter Homogenisierungseffekt wird erreicht, wenn die Rührscheibe einen Neigungswinkel zur Rotationsachse von 35 ° bis 60° haben. Die Rührscheiben rotieren bevorzugt um die gleiche Rotationsachse und in gleicher Rotationsgeschwindigkeit.

Wie erwähnt sind die Rührscheiben bevorzugt rund, was die Gefahr von Beschädigüngen empfindlicher Zellen zusätzlich vermindert. Unter bestimmten Umständen kann jedoch eine Gestaltung in Frage kommen, bei der der Rand der Scheiben nicht genau kreisförmig verläuft. In jedem Fall wird jedoch durch die rotierende Bewegung ein kreiszylinderförmiger Bereich des Kulturmediums erfäßt. Der Durchmesser dieses Bereichs wird als "effektiver Durchresser" der Rührscheiben Bezeichnet.

Die Bioreaktoren sind üblicherweise im wesentlichen kreiszylinderförmig mit einem abgerundeten Zylinderboden ausgebildet. Es hat sich als günstig erwiesen, wenn der effektive Durchmesser der Rührscheiben mindestens 40 %, bevorzugt mindestens 50 % und besonders bevorzugt mindestens 60 % des Innendurchmessers des Bioreaktors beträgt.

Die Rührscheiben können aus zahlreichen verschiedenen Materialien, insbesondere Kunststoffen oder ledierten Stählen hergestellt sein. Bevorzugt wird ein Material eingesetzt, an dem die kultivierten Zellen nicht haften. Bewährt hat sich insbesondere Teflon.

Die Erfindung wird im folgenden anhand eines in der Figur dargestellten Ausführungsbeispiels näher erläutert.

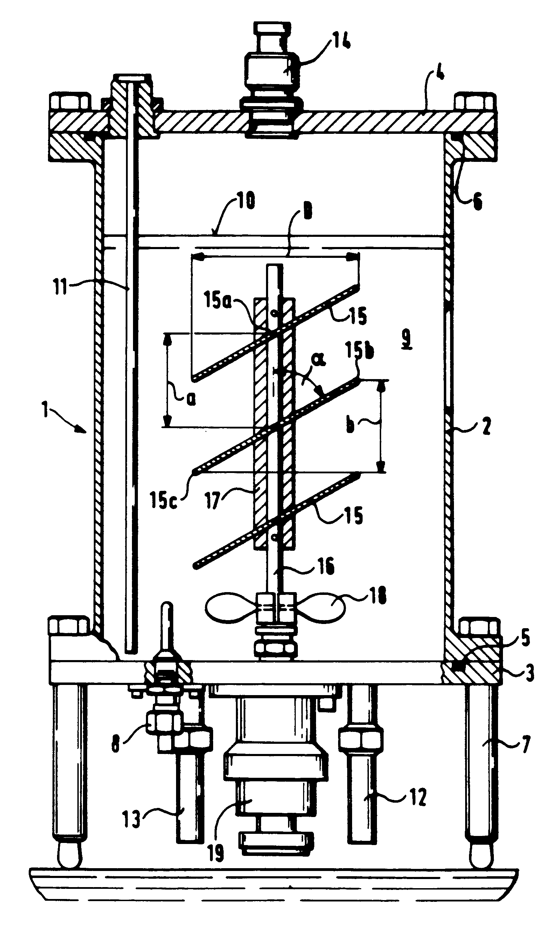

Figure 1 zeigt einen erfindungsgemäßen Bioreaktor im Querschnitt.

Der dargestellte Bioreaktor hat ein Gefäß 1 für Kulturmedium, das im wesentlichen aus einem zylinderförmigen Mittelteil 2, einem unteren Deckel 3 und einem oberen Deckel 4 besteht. Beide Deckel sind mit Flanschen mit dem Mittelteil 2 verschraubt und werden durch Dichtungen 5 bzw. 6 abgedichtet.

Das Gefäß 1 ruht auf Standsäulen 7. Eine Temperatursonde 8 ist so angeordnet, daß die Temperatur des Kulturmediums gemessen werden kann. Das Gefäß ist im Betrieb weitgehend mit dem Kulturmedium 9 gefüllt. Die Oberfläche des Kulturmediums 9 ist mit 10 bezeichnet.

Außer der Temperatursonde 8 sind weitere Einrichtungen zur Kontrolle der Umweltbedingungen in dem Kulturmedium vorgesehen. In der Figur dargestellt ist ein den Deckel 4 durchdringendes Tauchrohr 11, das beispielsweise für die Begasung des Kulturmediums mit Luft oder Sauerstoff eingesetzt werden kann. Im unteren Deckel 3 des Gefässes 1 sind Anschlußstutzen 12 bzw. 13 vorgesehen, durch die ein Temperierungsmedium zugeführt bzw. abgeführt werden kann, das in einer der Übersichtlichkeit halber nicht dargestellten Kühl- bzw. Heizschlange im Inneren des Gefässes 1 strömt, so daß zusammen mit der Temperatursonde 8 eine Temperaturregelung möglich ist.

Weitere Einrichtungen zur Kontrolle der Umweltbedingungen sind bei Bioreaktoren üblich, wurden jedoch der Übersichtlichkeit halber in der Zeichnung nicht dargestellt . Zur Beobachtung des IST-Zustandes des Kulturmediums werden beispielsweise Meßsonden zur Bestimmung des pH-Wertes, der Partialdrucke von O₂ und CO₂ und optische Fenster eingesetzt. Zur Steuerung der Umweltbedingungen in dem Kulturmedium 9 können beispielsweise Anschlüsse zur Zugabe bzw. Abfuhr von Substanzen (Gase, Nährstoffe, Abfallstoffe etc.) vorhanden sein. Der im Deckel 4 des dargestellten Fermenters erkennbare Mehrzweckanschlußstutzen 14 kann beispielsweise zu derartigen Zwecken dienen. Es kann aber auch ein Rückflußkühler mit Abluftfilter an dieser Stelle angeschlossen sein.

Die Erfindung richtet sich insbesondere auf die in dem dargestellten Biroreaktor eingesetzte Rühreinrichtung. Sie weist Rührscheiben 15 auf, die zentrisch und drehfest auf einer Rotationsachse 16 befestigt sind. Die Ebene der Rührscheiben 15 ist gegenüber der Rotationsachse um einen Neigungswinkel alpha geneigt. Die Befestigung der Rührscheiben 15 an der Rotationsachse 16 wird durch ein Bedfestigungsstück 17 verbessert. Zwischen der untersten Rührscheibe 15 und dem Boden des Gefässes 1 ist ein Schiffsschraubenrührer 18 ebenfalls drehfest auf der Rotationsachse 16 befestigt. Die Rotationsachse 16 wird von einem Rotationsantrieb 19 in üblicher Weise mit einer einstellbaren Drehzahl angetrieben.

Bei der Rotation der Rotationsachse 16 wird das Kulturmedium durch die umlaufenden Rührscheiben verdrängt und in eine komplizierte Bewegung versetzt. Einzelheiten dieser Bewegung können nicht angegeben werden. Praktische Experimente haben jedoch ergeben, daß mit Hilfe der erfindungsgemäßen schrägstehenden Rührscheiben eine überraschend schnelle und vollständige Homogenisierung des Kulturmediums 9 erreicht wird. Dabei werden bevorzugt mehrere Rührscheiben eingesetzt, deren Dimensionierung und Positionierung aufgrund der folgenden Hinweise vorgenommen werden kann.

Der Neigungswinkel alpha hat sich als verhältnismäßig unkritisch erwiesen. Winkel zwischen etwa 35 ° und etwa 60 °, führen zu einem guten Ergebnis. Anzahl und Neigung der Rührscheiben 15 sollten auf die Dimensionen des Gefässes 1 des Bioreaktors abgestimmt sein. Je höher der Bioreaktor ist, desto mehr Scheiben müssen eingesetzt werden. Dabei hängt der empfehlenswerte Abstand a zwischen benachbarten Rührscheiben von deren effektiven Durchmesser D und dem Neigungswinkel alpha ab. Dies dürfte sich daher erklären lassen, daß der von einer Rührscheibe erfaßte Bereich in Achsrichtung der Rotationsachse 16 umso größer ist, je kleiner der Neigungswinkel alpha und je größer der effektive Durchmesser D der Rührscheibe ist. Die gegenüber dem Zentrum am meisten in Achsrichtung versetzten Punkte des Rührscheibenrandes sind in der Figur mit 15b und 15c bezeichnet. Der von einer Rührscheibe erfaßte Bereich des Kulturmediums wird in Achsrichtung näherungsweise durch die umlaufenden Extrempunkte 15b bzw. 15c begrenzt, d. h. durch jeweils eine durch diese Punkte verlaufende, zur Achse 16 senkrechte Ebene. Es hat sich nun gezeigt, daß eine gute und effektive Durchmischung des Kulturmediums mit Rührscheiben erreicht wird, wenn der Abstand a benachbarter Rührscheiben höchstens etwa doppelt so groß und mindestens etwa halb so groß ist wie der Abstand b, der in Achsrichtung am meisten gegeneinander versetzten Extrempunkte des Rührscheibenrandes. Dieser Bereich vorteilhafter Plattenabstände ist erstaunlich groß und beweist die hervorragende Mischwirkung der Rührscheiben. Ganz besonderes gute Ergebnisse werden erreicht, wenn der Rührscheibenabstand a höchstens 50 % größer als der Abstand b ist.

Der Schiffsschraubenrührer 18 dient dazu, eventuell am Boden des Gefässess 1 sitzende Zellen aufzurühren, so daß sie vom Rührbereich der Rührscheiben 15 miterfaßt werden. Der Schiffsschraubenrührer 18 ist bevorzugt so gestaltet, daß er die Flüssigkeit bei der Rotation der Rotationsachse 16 nach unten wegdrückt. Statt des Schiffsschraubenrührers könnte auch ein anderes in der Nähe des Behälterbodens rotierendes oder umlaufendes Rührelement, be ispielsweise ein Rührpaddel eingesetzt werden.

Die in der Figur dargestellte Ausführungsform der Erfindung ist rotationssymetrisch aufgebaut, d. h. das Mittelteil 2 des Gefäßes 1, die Rührscheiben 15 und die Achse 16 sind jeweils bevorzugt kreisrund und koaxial zueinander. Ein derartiger rotationssymetrischer Aufbau ist besonders einfach und bei Bioreaktoren üblich. In besonderen Fällen kann jedoch eine Abweichung hiervon zweckmäßig sein, beispielsweise kann die Rotationsachse 16 gegenüber der zentralen Achse des Gefässes 1 versetzt sein. In diesem Fall gelten die auf einen rotationssymetrischen Aufbau bezogenen Dimensionierungsangaben entsprechend.

Die Rührscheiben sind bei dem dargestellten Ausführungsbeispiel alle gleich groß und in gleichmäßigem Abstand parallel zueinander jeweils drehfest auf einer gemeinsamen Rotationsachse befestigt. Dieser Aufbau ist einfach und kostengünstig. Aus besonderen Gründen kann es jedoch auch zweckmäßig sein, die schräggestellten Rührscheiben in anderer Weise einzusetzen, beispielsweise in einer Anordnung, bei der sie zwar auf einer gemeinsamen Achse sitzen, jedoch nicht parallel zueinander verlaufen, oder bei der sie sogar auf verschiedenen (zueinander koaxialen oder nicht koaxialen) Achsen sitzen, so daß auch ihre Rotationsgeschwindigkeit verschieden sein kann.

## Patentansprüche

1. Bioreaktor zum Kultivieren von vermehrungsfähigem biologischem Material, insbesondere von auf Mikrocarriern haftenden adherenten Zellen, mit eines Gefäß für ein Kulturmedium (9) mit Einrichtungen zur Kontrolle der Umweltbedingungen (8, 11, 12, 13) in des Kulturmedium und einer Rühreinrichtung zur homogenen Verteilung der Zellen in des Kulturmedium, welche einen Rotationmantrieb (19) und eine im Inneren dem Gefäßes verlaufende Rotationsachse (16) aufweist, dadurch gekennzeichnet, daß
die Rühreinrichtung mehrere an der Rotationsachse (16) befestigte, zur Rotationsachse geneigte flache Rührscheiben (15) aufweist.

2. Bioreaktor nach Anspruch 1, dadurch gekennzeichnet, daß der Neigungswinkel (alpha der Rührscheibe (15) zur Rotationsachse (16) 35 ° - 60 ° beträgt.

3. Bioreaktor nach einem der vorhergehenden Ansprüche, dadurch gekennzeichnet, daß der Abstand (a) benachbarter Rührscheiben (15) höchstens etwa doppelt so groß und mindestens etwa halb so groß ist, wie der Abstand (b) der in Achsrichtung am meiste gegeneinander versetzten Extrempunkte (15b, 15c) des Rührscheibenrandes.

4. Bioreaktor nach einem der vorhergehenden Ansprüche, dadurch gekennzeichnet, daß der effektive Durchmesser (D) der Rührscheiben (15) mindestens 40 %, bevorzugt mindestens 50 %, besonders bevorzugt mindestens 60 % des Innendurchmessers des Behälters beträgt.

5. Bioreaktor nach einem der vorhergehenden Ansprüche, dadurch gekennzeichnet, daß die Rühreinrichtung zusätzlich ein in der Nähe den Behälterbodens rotierenden oder umlaufendes Rührelement, insbesondere einen Schiffsschraubenrührer (18) aufweist.

6. Bioreaktor nach einem der vorhergehenden Ansprüche, dadurch gekennzeichnet, daß das Material der Rührscheiben (15) so gewählt ist, daß das Biomaterial daran nicht haftet.

7. Bioreaktor nach einem der vorhergehenden Ansprüche, dadurch gekennzeichnet, daß der Rotationsantrieb (19) so ausgelegt ist, daß die Rotationsachse mit einer Umdrehungszahl von weniger als 50 pro Minute, bevorzugt weniger als 35 pro Minute, besonders bevorzugt weniger als 20 pro Minute gedreht wird.

## Claims

1. Bioreactor for culturing biological material capable of reproducing, particularly aggregating cells which adhere to microcarriers, comprising a vessel for a culturing medium (9) provided with devices for controlling the evironmental conditions (8, 11, 12, 13) in the culturing medium and a stirring device for homogeneously distributing the cells in the culture medium, said stirring device being provided with a rotary drive (19) and a rotary axle disposed in the interior of the vessel (16), characterized in that
the stirring device is provided with several flat stirring disks (15) mounted to the rotary axle (16) and inclined toward said axle.

2. Bioreactor in accordance with claim 1, characterized in that the angle of inclination (alpha) formed between the stirring disk (15) and the axle of rotation (16) is 35°-60°C.

3. Bioreactor in accordance with one of the aforesaid claims, characterized in that distance (a) between adjacent stirring disks (15) is not more than approximately twice and not less than approximately half the length of distance (b) between the outermost points (15b, 15c) of a stirring disk in axial direction.

4. Bioreactor in accordance with one of the aforesaid claims, characterized in that the effective diameter (D) of the stirring disks (15) is at least 40%, preferably at least 50%, more preferably at least 60% of the internal diameter of the vessel.

5. Bioreactor in accordance with one of the aforesaid claims, characterized in that the stirring device is provided with an additional stirring element, particularly a propeller-type stiffer (18), rotating or turning in the vicinity of the vessel bottom.

6. Bioreactor in accordance with one of the aforesaid claims, characterized in that the material of the stirring disks (15) is selected such that the biomaterial does not adhere to it.

7. Bioreactor in accordance with one of the aforesaid claims, characterized in that the rotary drive (19) is designed such that the rotating axle turns with less than 50 revolutions per minute, preferably less than 35 revolutions per minute, more preferable less than 20 revolutions per minute.

## Revendications

1. Bioréacteur pour la culture de matériel biologique capable de se multiplier, en particulier des cellules adhérentes qui adhèrent à des microcarriers qui comprend un récipient pour un milieu de culture (9) équipé de dispositifs pour le contrôle des conditions environnantes (8, 11, 12, 13) dans le milieu de culture et d'un dispositif de mélange pour la répartition homogène des cellules dans le milieu de culture, qui présente un entrainement par rotation (19) et un axe de rotation (16) à l'intérieur du récipient, caractérisé en ce que
le dispositif de mélange présente plusieurs disques mélangeurs plats (15) inclinés vers l'axe de rotation (16) et fixés à ce dernier.

2. Bioréacteur selon la revendication 1, caractérisé en ce que l'angle d'inclination (alpha) entre le disque mélangeur (15) et l'axe de rotation (16) est de 35° à 60°.

3. Bioréacteur selon l'une des revendications précédentes, caractérisé en ce que la distance (a) entre deux disques mélangeurs avoisinants (15) est au maximum environ le double et au minimum environ la moitié de la distance (b) entre les points extrêmes (15b, 15c) du bord d'un disque mélangeur dans le sens de l'axe.

4. Bioréacteur selon l'une des revendications précédentes, caractérisé en ce que le diamètre effectif (D) du disque mélangeur comporte au moins 40%, de préférence au moins 50% et de toutes préférence au moins 60% du diamètre intérieur du récipient.

5. Bioréacteur selon l'une des revendications précédentes, caractérisé en ce que le dispositif de mélange présente en complément un élément mélangeur, en particulier une hélice mélangeuse (18) qui effectue un mouvement rotatif ou tournant à proximité du fond du récipient.

6. Bioréacteur selon l'une des revendications précédentes, caractérisé en ce que le matériau des disques mélangeurs (15) est choisi de sorte que le matériel biologique n'y adhère pas.

7. Bioréacteur selon l'une des revendications précédentes, caractérisé en ce que le mouvement de rotation (19) est conçu de sorte que l'axe de rotation tourne avec un nombre de tours de moins de 50 tours/mn, de préférence de moins de 35 tours/mn, de toute préférence de moins de 20 tours/mn.
